# EUROPEAN PATENT SPECIFICATION

(11) **EP 4 080 219 B1**
(45) Date of publication and mention of the grant of the patent: **20.11.2024**
(21) Application number: 22168390.7
(22) Date of filing: 14.04.2022
(51) Int. Cl.: G01N 33/84, G01N 33/53, G01N 33/68, G01N 33/569, C12N 15/115

(54) **CONJUGATE**
KONJUGAT
CONJUGUÉ

(30) Priority: 20.04.2021 JP 2021071056; 27.12.2021 JP 2021213138
(43) Date of publication of application: 26.10.2022
(73) Proprietor: DENSO CORPORATION, Aichi-pref., 448-8661 (JP)
(72) Inventor: NUKAZUKA, Akira, Kariya-city, Aichi-pref., 448-8661 (JP); ASANO, Mana, Kariya-city, Aichi-pref., 448-8661 (JP); HAYAKAWA, Kei, Kariya-city, Aichi-pref., 448-8661 (JP); YOSHIMITSU, Yuuya, Kariya-city, Aichi-pref., 448-8661 (JP); KANO, Kazuhiko, Kariya-city, Aichi-pref., 448-8661 (JP); NAKAGAWA, Kazuhisa, Kariya-city, Aichi-pref., 448-8661 (JP); NIIMOTO, Mai, Kariya-city, Aichi-pref., 448-8661 (JP)
(74) Representative: Winter, Brandl - Partnerschaft mbB

(56) References cited:
- WO-A1-2020/039201
- WO-A1-2021/053206
- US-A1- 2015 031 014
- US-B2- 9 964 538
- WARSINKE AXEL ET AL: "TOWARDS SEPARATION-FREE ELECTROCHEMICAL AFFINITY SENSORS BY USING ANTIBODIES, APTAMERS, AND MOLECULARLY IMPRINTED POLYMERS - A REVIEW", ANALYTICAL LETTERS, TAYLOR & FRANCIS INC, US, vol. 39, no. 13, 2 February 2007 (2007-02-02), pages 2507 - 2556, XP008073138, ISSN: 0003-2719, DOI: 10.1080/00032710600853903
- YANLING SONG ET AL: "Discovery of Aptamers Targeting the Receptor-Binding Domain of the SARS-CoV-2 Spike Glycoprotein", ANALYTICAL CHEMISTRY, vol. 92, no. 14, 18 June 2020 (2020-06-18), US, pages 9895 - 9900, XP055759814, ISSN: 0003-2700, DOI: 10.1021/acs.analchem.0c01394
- SONG YANLING ET AL: "Supporting Information Discovery of Aptamers Targeting the Receptor-Binding Domain of the SARS- CoV-2 Spike Glycoprotein Competition assay of CoV2-RBD-1C and CoV2-RBD-4C aptamers assay of CoV2-RBD-1C and CoV2-RBD-4C aptamers", 17 June 2020 (2020-06-17), pages 1 - 6, XP055956423, Retrieved from the Internet <URL:https://pubs.acs.org/doi/10.1021/acs.analchem.0c01394?goto=supporting-info> [retrieved on 20220831]
- KATSU TAKASHI ET AL: "Construction of a p-nitrophenolate-sensitive membrane electrode and its application to an enzyme assay.", CHEMICAL AND PHARMACEUTICAL BULLETIN, vol. 35, no. 9, 30 September 1987 (1987-09-30), JP, pages 3922 - 3924, XP055956053, ISSN: 0009-2363, Retrieved from the Internet <URL:https://www.jstage.jst.go.jp/article/cpb1958/35/9/35_9_3922/_pdf/-char/en> DOI: 10.1248/cpb.35.3922
- MIAO LUYANG ET AL: "pH Readout enhanced ELISA for point-of-care testing of cardiac troponin I", CHINESE CHEMICAL LETTERS, ELSEVIER, AMSTERDAM, NL, vol. 28, no. 9, 28 April 2017 (2017-04-28), pages 1878 - 1880, XP085174477, ISSN: 1001-8417, DOI: 10.1016/J.CCLET.2017.04.018
- ZHANG YUN ET AL: "Enhanced ELISA using a handheld pH meter and enzyme-coated microparticles for the portable, sensitive detection of proteins", CHEMICAL COMMUNICATIONS, vol. 52, no. 17, 9 December 2015 (2015-12-09), UK, pages 3474 - 3477, XP055955722, ISSN: 1359-7345, DOI: 10.1039/C5CC09852A
- SOHEE JEONG ET AL: "Sensitivity and Selectivity on Aptamer-Based Assay: The Determination of Tetracycline Residue in Bovine Milk", THE SCIENTIFIC WORLD JOURNAL, vol. 2012, 1 April 2012 (2012-04-01), pages 1 - 10, XP055366168, DOI: 10.1100/2012/159456
- PLATELLA CHIARA ET AL: "G-quadruplex-based aptamers against protein targets in therapy and diagnostics", BIOCHIMICA ET BIOPHYSICA ACTA, ELSEVIER, AMSTERDAM, NL, vol. 1861, no. 5, 16 November 2016 (2016-11-16), pages 1429 - 1447, XP085056309, ISSN: 0304-4165, DOI: 10.1016/J.BBAGEN.2016.11.027
- KIM YEON-JUN ET AL: "Enzyme-linked aptamer-based sandwich assay (ELASA) for detecting Plasmodium falciparum lactate dehydrogenase, a malarial biomarker", RSC ADVANCES, vol. 12, no. 45, 1 January 2022 (2022-01-01), GB, pages 29535 - 29542, XP093171863, ISSN: 2046-2069, DOI: 10.1039/D2RA03796C

## Description

### TECHNICAL FIELD

The present disclosure relates to a kit of parts comprising a conjugate and a pH meter.

### BACKGROUND

In technical fields such as biotechnology and healthcare, a method for detecting a target substance in a sample is used. As such detection method, Enzyme-Linked ImmunoSorbent Assay (i.e., ELISA) has been known. The target substance is a protein or the like.

In the enzyme-linked immunosorbent assay, a conjugate of an immunoglobulin and a label is bound to the target substance. Such conjugate is described in Biosensors and Bioelectronics 117 (2018) 175-182. As the immunoglobulin, monoclonal antibodies, polyclonal antibodies and the like are known.

WARSINKE AXEL ET AL, "TOWARDS SEPARATION-FREE ELECTROCHEMICAL AFFINITY SENSORS BY USING ANTIBODIES, APTAMERS, AND MOLECULARLY IMPRINTED POLYMERS - A REVIEW", ANALYTICAL LETTERS, TAYLOR & FRANCIS INC, US, Vol. 39, No. 13, January 1, 2006, is directed to electrochemical affinity sensors using antibodies and aptamers and gives an overview of the main recognition elements (antibodies, aptamers, and molecularly imprinted polymers) and measuring principles.

WO 2020 039201 A is directed to apparatus and methods of detecting and/or quantifying a target moiety in a sample comprising the use of a nucleic-acid molecule based recapture event.

WO 2021 053206 A discloses a method for detecting an analyte in a liquid sample using immunoassays in combination with an electrical readout by detecting an electric potential difference and/or a change in an electric current between a working electrode and a reference electrode.

US 9 964 538 B2 discloses a sandwich immune assay where the conversion of a substrate by a reporter enzyme is read out via mass spectrometry.

US 2015/031014 A is directed to a method and sensors for detecting a broad array of target molecules, such as nucleic acids (e.g., DNA and RNA), proteins, toxins, pathogens, cells, and metals with a pH meter by using pH-change as read-out upon target-binding to a recognition molecule.

The discovery of an aptamer against the receptor-binding domain of the SARSCoV-2 Spike glycoprotein, its use as diagnostic probes (PG 9899) and an aptamer labelled with FAM (fluorescein) are reported in YANLING SONG ET AL, "Discovery of Aptamers Targeting the Receptor-Binding Domain of the SARS-CoV-2 Spike Glycoprotein", ANALYTICAL CHEMISTRY, US, Vol. 92, No. 14, June 18, 2020; and Song Yanling ET AL, "Supporting Information Discovery of Aptamers Targeting the Receptor-Binding Domain of the SARS- CoV-2 Spike Glycoprotein Competition assay of CoV2-RBD-1C and CoV2-RBD-4C aptamers assay of CoV2-RBD-1C and CoV2-RBD-4C aptamers", June 17, 2020, pages 1-6.

KATSU TAKASHI ET AL, "Construction of a p-nitrophenolate-sensitive membrane electrode and its application to an enzyme assay.", CHEMICAL AND PHARMACEUTICAL BULLETIN, JP, Vol. 35, No. 9, January 1, 1987, pages 3922-3924, discloses a p-nitrophenolate-sensitive membrane electrode for measuring enzyme activity of alkaline phosphatase, the use of p-nitrophenolate in colorimetry and its application to an enzyme immunoassay.

MIAO LUYANG ET AL, "pH Readout enhanced ELISA for point-of-care testing of cardiac troponin I", CHINESE CHEMICAL LETTERS, ELSEVIER, AMSTERDAM, NL, (20170428), Vol. 28, No. 9, April 28, 2017, pages 1878-1880, describes a pH Readout enhanced ELISA using synthetic melanin nanoparticles (SMNPS) for co-immobilization of glucose oxidase (GOx) and a second antibody (Ab₂) as signal labels, and a portable pH meter as signal readout device for point-of-care testing (POCT) of cardiac troponin I.

ZHANG YUN ET AL, "Enhanced ELISA using a handheld pH meter and enzyme-coated microparticles for the portable, sensitive detection of proteins", CHEMICAL COMMUNICATIONS, UK, (20151209), Vol. 52, No. 17, December 9, 2015, pages 3474-3477, discloses an ELISA where instead of reading out an enzyme catalyzed color reaction, an enzyme catalyzed change in pH is measured via a handheld pH meter, the enzyme being glucose oxidase and the pH-change being due to the reaction of glucose into gluconic acid.

SOHEE JEONG ET AL, "Sensitivity and Selectivity on Aptamer-Based Assay: The Determination of Tetracycline Residue in Bovine Milk", THE SCIENTIFIC WORLD JOURNAL, (20120401), Vol. 2012, January 1, 2012, pages 1-10, report on a competitive enzyme-linked aptamer assay (ELAA) with an DNA- and RNA-aptamer for detecting tetracycline residue in bovine milk.

PLATELLA CHIARA ET AL, "G-quadruplex-based aptamers against protein targets in therapy and diagnostics", BIOCHIMICA ET BIOPHYSICAACTA, ELSEVIER, AMSTERDAM, NL, (20161116), Vol. 1861, No. 5, November 16, 2016, pages 1429-1447, provides an overview about G4 based aptamers and their therapeutic potential.

### SUMMARY

In the sample, the target substance may exist close to other substances. For example, when a plurality of target substances form a complex or are present at a high density on an outer envelope such as a cell membrane, the target substance exists close to the adjacent target substance. Further, when the target substance is present on the outer envelope such as a cell membrane and another giant substance is present in the vicinity of the target substance, the target substance is close to the giant substance.

The size of the immunoglobulin is large. Therefore, when the target substance is close to another substance, the conjugate including the immunoglobulin is interfered by the other substance and may not be able to bind to the target substance. As a result, analytical sensitivity to the target substance is deteriorated.

In one aspect of the present disclosure, it is preferable to provide a detection method in which analytical sensitivity is not deteriorated even when the target substance is close to another substance.

The present disclosure relates to a kit of parts for detecting a target substance in a sample comprising
a conjugate including a binding substance having an activity to bind to a target substance and a label causing a detectable phenomenon, and
a pH meter,
wherein
the target substance is SARS-CoV-2,
the binding substance is a nucleic acid aptamer,
the detectable phenomenon is decrease of pH that can be detected with the pH meter, and
the label is an alkaline phosphatase that metabolises pNPP resulting in the decrease of pH.

A molecular weight of the binding substance is preferably less than a molecular weight of an immunoglobulin.

The binding substance of the conjugate of the present disclosure preferably has a molecular weight less than that of an immunoglobulin. Therefore, the size of the conjugate, which is one aspect of the present disclosure, is smaller than that of the conjugate including the immunoglobulin. Therefore, even when the target substance is close to another substance, the conjugate of the present disclosure is less likely to be interfered by the other substance and can easily bind to the target substance. As a result, using the conjugate of the present disclosure, the analytical sensitivity to the target substance can be improved.

### BRIEF DESCRIPTION OF DRAWINGS

FIG. 1 is an explanatory diagram illustrating a structure of a conjugate and a method for detecting a target substance.
FIG. 2 is an explanatory diagram illustrating steps in synthesizing the conjugate.
FIG. 3 is an explanatory diagram illustrating a metabolism caused by an enzymatic activity of alkaline phosphatase.
FIG. 4 is an explanatory diagram illustrating steps of the detection method.
FIG. 5 is a table of measurement results of absorbance.
FIG. 6 is an explanatory diagram illustrating steps of the detection method.
FIG. 7 is a table of measurement results of pH.
FIG. 8 is an explanatory diagram illustrating steps in a test for confirming an expression of a function of a label.
FIG. 9 is a table of measurement results of pH.

### DESCRIPTION OF EMBODIMENTS

Exemplary embodiments of the present disclosure will be described with reference to the drawings.

### 1. Conjugate

### (1-1) Composition of a conjugate

The conjugate includes a binding substance. The binding substance has an activity to bind to a target substance (hereinafter, referred to as a binding activity). The binding substance has a binding activity even when fused with a label.

The binding substance is a nucleic acid aptamer. The molecular weight of the binding substance is preferably less than the molecular weight of an immunoglobulin.

The number of bases of the nucleic acid aptamer falls within a range, for example, between 10, inclusive, and 100, inclusive. When the number of bases is 100 or less, even when the target substance is close to another substance, the conjugate is less likely to be interfered by the other substance and easily binds to the target substance.

The nucleic acid aptamer may be one in which a plurality of units each consisting of nucleic acids are linked to each other, for example. The number of bases in each of the units preferably falls within a range between 10, inclusive, and 100, inclusive. When the number of bases is 100 or less, even when the target substance is close to another substance, the conjugate is less likely to be interfered by the other substance and easily binds to the target substance.

Examples of the nucleic acid aptamer include a DNA aptamer and an RNA aptamer. The binding substance can be chemically synthesized, for example, by an in vitro process.

The binding substance binds to the target substance, for example, by an intramolecular interaction like an antigen-antibody interaction. Bonds by the intermolecular interaction like an antigen-antibody interaction include hydrogen bonds, bonds by electrostatic complementarity, bonds by hydrophobic contact, steric bonds and the like.

The conjugate includes a label. The label is fused with the binding substance. The label exhibits a function. When the label exhibits its function, a detectable phenomenon occurs. According to the present invention, the label is an alkaline phosphatase that metabolises pNPP resulting in the decrease of pH.

For example, the binding substance and the label are fused with each other through a chemical substituent or a nucleic acid binding protein. For example, each of the label and the binding substance includes a chemical substituent or a nucleic acid binding protein. For example, the label is fused with the binding substance by a binding between the chemical substituent or the nucleic acid binding protein of the label and the chemical substituent or the nucleic acid binding protein of the binding substance.

The chemical substituent contained in the label is at least one selected from the group consisting of biotin, primary amine, azide, alkyne, dibenzocyclooctyne, bicyclononyne, 2'-propynyl, 2'-O-propargyl, thiol, avidin, streptavidin, neutravidin, N-hydroxysuccinimide, maleimide and 5-halouracil. Examples of 5-halouracil include 5-iodouracil, 5-bromouracil and the like. 5-halolasyl is a chemical substituent that can form cross-linking with UV.

The nucleic acid-binding protein contained in the label is at least one selected from the group consisting of zinc finger and CRISPR.

The chemical substituent contained in the binding protein is at least one selected from the group consisting of biotin, primary amine, azide, alkyne, dibenzocyclooctyne, bicyclononyne, 2'-propynyl, 2'-O-propargyl, thiol, avidin, streptavidin, neutravidin, N-hydroxysuccinimide, maleimide, 5-halouracil. Examples of 5-halouracil include 5-iodouracil, 5-bromouracil and the like. 5-halolasyl is a chemical substituent that can form cross-linking with UV.

The nucleic acid-binding protein contained in the binding substance is at least one selected from the group consisting of zinc finger and CRISPR.

The chemical substituent contained in the label and the chemical substituent contained in the binding substance are different. For example, when the chemical substituent contained in the label is biotin, the chemical substituent contained in the binding substance is avidin, streptavidin or neutravidin.

In the conjugate of the present invention, the target substance is SARS-CoV-2. The binding is achieved via an antigen contained in the virus, including RBD region (hereinafter referred to as RBD) of the novel coronavirus SARS-CoV-2. Examples of the virus include the novel coronavirus SARS-CoV-2 and the like.

### (1-2) Advantages of the conjugate

The conjugate of the present disclosure has the following advantages.

(1A) A plurality of target substances may form a complex. Examples of the complex include a dimer, a trimer, a tetramer and the like. In one complex, each target substance is close to another target substance.

Due to the small size of the conjugate, when the conjugate approaches the target substance, the conjugate is less likely to be interfered by the adjacent target substance. Therefore, the conjugates can bind to each of the target substances forming the complex. As a result, the conjugate of the present disclosure can improve analytical sensitivity to the target substance.

Proteins in living organisms often form complexes. Examples of proteins in living organisms include a membrane protein and an envelope protein. Examples of the membrane protein include a receptor protein and the like. Examples of the envelope protein include a viral spike protein and the like. By using the conjugate of the present disclosure, it is possible to improve the analytical sensitivity to the protein in living organisms even when the protein forms a complex.

(1B) A plurality of target substances may be present at high density on an outer envelope such as a cell membrane. In this case, each of the target substances are very close to the adjacent target substance.

Due to the small size of the conjugate, when the conjugate approaches the target substance, the conjugate is less likely to be interfered by the adjacent target substance. Therefore, the conjugates can bind to each of the target substances present at high density. As a result, the conjugate of the present disclosure can improve analytical sensitivity to the target substance.

Proteins in living organisms are often present at high density on the cell membrane. Examples of a protein in living organisms include the proteins mentioned in (1A). The conjugate of the present disclosure can improve the analytical sensitivity to proteins in living organisms even when the proteins present at a high density on the cell membrane.

(1C) The target substance may be present on an outer envelope such as a cell membrane, and another giant substance may be present in the vicinity of the target substance.

Due to the small size of the conjugate, the conjugate is less likely to be interfered by the giant substance when approaching the target substance. Therefore, the conjugate can bind to the target substance existing in the vicinity of the giant substance. As a result, the conjugate of the present disclosure can improve analytical sensitivity to the target substance.

A protein in living organisms is present on the outer envelope such as a cell membrane and the like, and a giant substance is often present in the vicinity of the protein. Examples of a protein in living organisms include the proteins mentioned in (1A). The conjugate of the present disclosure can improve the analytical sensitivity to the protein in living organisms even when a giant substance is present in the vicinity of the protein.

(1D) The epitope of the target substance may change due to a mutation and the like. When the epitope of the target substance changes, the current binding substance may lose its binding activity. In particular, when the target substance is a protein expressed by an RNA virus such as influenza virus or coronavirus, mutation occurs frequently and the epitope changes frequently.

The binding substance of the conjugate of the present disclosure can be chemically synthesized, for example, by an in vitro process. Since the binding substance can be chemically synthesized by an in vitro process, it is easier to develop a binding substance to have a binding activity to the target substance having a changed epitope as compared with the case of synthesizing a monoclonal antibody or a polyclonal antibody.

### 2. Method for detecting the target substance

### (2-1) Procedure of detection method of the target substance

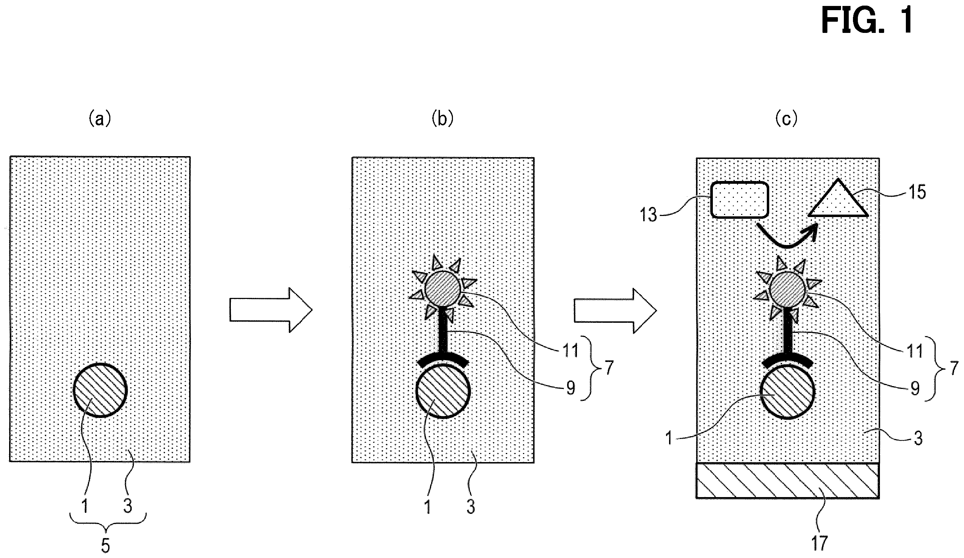

The method for detecting the target substance can be performed, for example, by the following procedure.
(i) As shown in (a) of FIG. 1, the target substances 1 are prepared. In FIG. 1, only one target substance 1 is illustrated for descriptive purpose. The target substances 1 are present in, for example, a solution 3. The target substances 1 and the solution 3 form a sample 5.
(ii) Next, as shown in (b) of FIG. 1, a treatment to bind at least some of the conjugates 7 to the target substances 1 is performed. For example, by allowing the conjugates 7 and the target substances 1 to coexist in the solution 3, at least some of the conjugates 7 bind to the target substances 1.
   Each of the conjugates 7 includes a binding substance 9 and a label 11. The conjugate 7 is the one described in the above section "1. Conjugate". The binding substances 9 of some of the conjugates 7 are bound to the target substances 1 to form coupled bodies.
(iii) Next, among the conjugates 7, unbound conjugates 7 that are not bound to the target substances 1 are removed.
(iv) Next, the function of the label 11 of the conjugate 7 is expressed. By expressing the function of the label 11, a detectable phenomenon occurs. According to the present invention, the function of the label 11 is an enzymatic activity.
   The enzymatic activity may be a metabolism starting from a substrate 13 as shown in FIG. 1 (c). The metabolism starting from the substrate 13 produces a product 15. The product 15 may be hydrogen ions. Alternatively, the product 15 consumes hydrogen ions. Therefore, the metabolism starting from the substrate 13 may generate or consume hydrogen ions. When the metabolism that generates or consumes hydrogen ions occurs, the hydrogen ion concentration in the solution 3 changes. The change in the hydrogen ion concentration corresponds to a detectable phenomenon caused by the function of the label 11.
(v) Next, the label 11 is detected. Since the conjugates 7 are bound to the target substances 1, detecting the label 11 is equivalent to detecting the target substances 1.

In order to detect the label 11, the phenomenon caused by the function of the label 11 is observed. If the phenomenon can be observed, it means that the label 11 can be detected.

According to the present invention, the phenomenon caused by the label is a change in a concentration of an ion such as generation, consumption or absorption of hydrogen ion, potassium ion, sodium ion, calcium ion, lithium ion, ammonium ion or chloride ion, the phenomenon can be detected by pH meter, ion sensor or ion-sensitive electric field effect transistor.

For example, when the function of the label 11 is an enzymatic activity that induces a metabolism that generates, consumes or absorbs hydrogen ions, change amount of hydrogen ion concentration is measured with a measuring instrument 17 shown in FIG. 1 (c). When there is a change in the hydrogen ion concentration, it means that the label 11 is detected.

### (2-2) Advantages of the method for detecting the target substance

The method for detecting a target substance of the present disclosure achieves the advantages (1A) to (1D) described above, and further achieves the following advantages.

(2A) According to the method for detecting a target substance in the present disclosure, the concentration of the target substances in the sample can be easily and highly sensitively measured.

(2B) As the function of the label is an enzymatic activity that induces a metabolism that generates, consumes or absorbs hydrogen ions, the target substances can be detected by a change in the hydrogen ion concentration in the solution.

According to the method for detecting the target substance disclosed in the present disclosure, only the conjugates bound to the target substances induce a metabolism that produces, consumes or absorbs hydrogen ions. Therefore, the higher the concentration of the target substances, the greater the amount of change in the hydrogen ion concentration in the solution. Therefore, according to the method for detecting the target substance in the present disclosure, the concentration of the target substances can be quantified based on the amount of change in the hydrogen ion concentration in the solution.

### 3. Examples

### (3-1) Synthesis of conjugate

A DNA aptamer having the base sequence of SEQ ID NO: 1 (hereinafter, referred to as an unmodified DNA aptamer) was chemically synthesized by an in vitro process. The base sequence of the unmodified DNA aptamer is "5'-CAGCACCGAC CTTGTGCTTT GGGAGTGCTG GTCCAAGGGC GTTAATGGAC A-3'". The unmodified DNA aptamer is described in Anal. Chem. 2020, 92, 9895-9900 (hereinafter, referred to as Reference 1).

According to the description in Reference 1, the unmodified DNA aptamer is a DNA aptamer whose target substance is RBD of the spike glycoprotein of the novel coronavirus SARS-CoV-2. Next, 5' end of the unmodified DNA aptamer was chemically modified with biotin via a C6 spacer.

Through the above steps, a DNA aptamer was obtained. The DNA aptamer corresponds to a binding substance. The DNA aptamer was dissolved in a phosphate buffer solution (hereinafter, referred to as 1×PBS/T) to prepare a DNA aptamer solution. The concentration of the DNA aptamer in the DNA aptamer solution was 10 µmol/l.

1xPBS/T contained 0.05% (v/v) of the surfactant Tween 20. In addition, 1xPBS/T contained 137 mmol/l of NaCl, 8.1 mmol/l of Na₂HPO₄, 2.7 mmol/l of KCl and 1.47 mmol/l of KH₂PO₄.

As a label, a streptavidin-alkaline phosphatase conjugate (produced by Thermo Fisher Scientific Inc., product number S921) was prepared.

This label is an enzyme. The streptavidin-alkaline phosphatase conjugate is obtained by modifying alkaline phosphatase with streptavidin. Streptavidin is a chemical substituent that modifies the label. The label was dissolved in 1xPBS/T to prepare a label solution. The concentration of the label in the label solution was 20 µmol/l.

100 µL of the DNA aptamer solution and 5 µL of the label solution were mixed and let stand at room temperature for 1 hour. At this time, the DNA aptamer and the label were fused by the interaction between biotin-streptavidin, and the conjugate was synthesized.

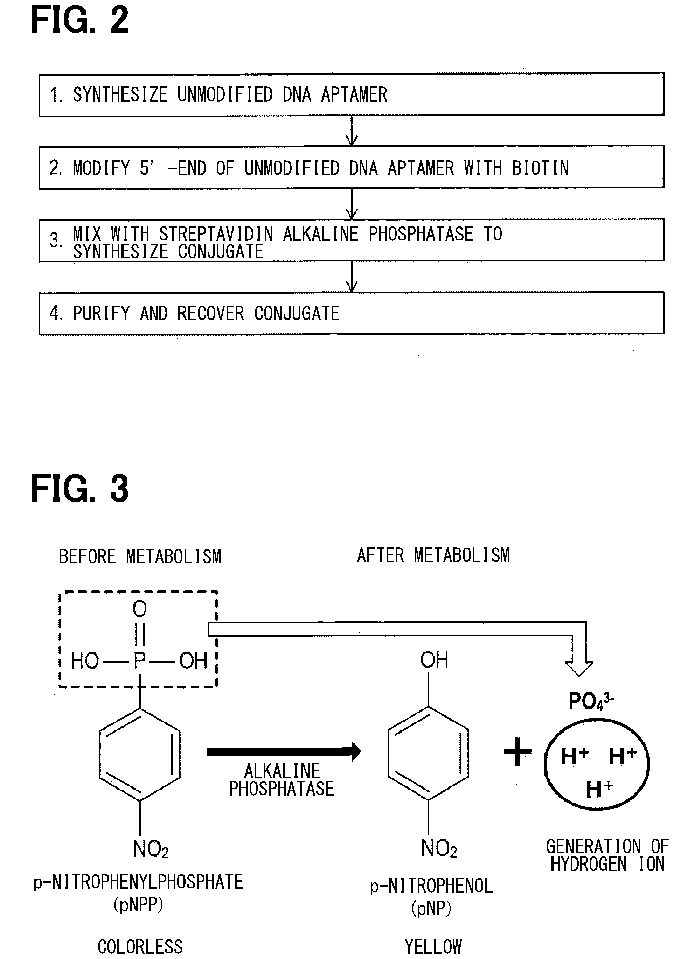

The conjugate was then purified and recovered. Specifically, the DNA aptamer that is not fused with the label was separated from the conjugate using an ultrafiltration filter to recover the conjugate. The flow of the process of synthesizing the conjugate is shown in FIG. 2.

The binding substance may be a binding substance other than the above-mentioned DNA aptamer. The binding substance may be, for example, a low-molecular weight protein formulation or an RNA aptamer. Examples of the low-molecular weight protein formulation include fragment antibody, single chain antibody, Diabody, Nanobody, VHH, peptide aptamer and the like.

Further, the base sequence of the DNA aptamer may be a base sequence other than the base sequence of SEQ ID NO: 1. The base sequence of the DNA aptamer can be selected according to the target substance.

Further, the fusion of the binding substance and the label may be a fusion other than the fusion based on the biotin-streptavidin interaction. When the nucleic acid aptamer is used as a binding substance, the end of the nucleic acid aptamer other than the 5' end may be fused with the label.

When the low-molecular weight protein formulation is used as a binding substance, the N-terminal or C-terminal of the low-molecular weight protein formulation may be fused with the label.

### (3-2) Using method for detecting the target substance

As a target substance, Spike S1-His Recombinant Protein (produced by Sino Biological Inc., product number 4059-V08H) is prepared. This target substance is hereinafter referred to as S1. The amino acid sequence of S1 includes RBD.

S1 was dissolved in 0.1 M carbonate buffer to prepare an S1-A solution and an S1-B solution. The concentration of S1 in the S1-A solution was 5 µg/mL. The concentration of S1 in the S1-B solution was 1 µg/mL. The pH of the 0.1 M carbonate buffer was adjusted to 9.6.

In addition, α-amylase (produced by Lee Biosolutions, Inc., product number 120-17) was prepared as a target substance for a negative control. α-Amylase was dissolved in 0.1 M carbonate buffer to prepare an α-amylase solution. The concentration of α-amylase in the α-amylase solution was 5 µg/mL.

The buffer solution used for preparing the S1-A solution, the S1-B solution, and the α-amylase solution may be a buffer solution other than 0.1 M carbonate buffer solution described above. Examples of the buffer solution include commonly used buffer solutions such as 1xPBS/T, 1xPBS, 1xTBS/T and 1xTBS.

Next, 100 µL of S1-A solution was added dropwise onto a part of the ELISA well of the 96-well plate H type for ELISA (produced by Sumitomo Bakelite Co., Ltd., product number MS-8896F), and it was let stand at 4 °C overnight. Hereinafter, the part of the ELISA well onto which the S1-A solution was dropped is referred to as a S1-A well. At this time, S1 was immobilized on the S1-A well.

Further, 100 µL of the S1-B solution was added dropwise onto a part of the ELISA well other than the S1-A well, and it was let stand at 4 °C overnight. Hereinafter, the part of the ELISA well onto which the S1-B solution is dropped will be referred to as an S1-B well. At this time, S1 was immobilized on the S1-B well.

Further, 100 µL of the α-amylase solution was added dropwise onto a part of the ELISA well, which was neither S1-A well nor S1-B well, and it was let stand at 4 °C overnight. Hereinafter, the part of the ELISA well onto which the α-amylase solution is added dropwise will be referred to as an α-amylase well. At this time, α-amylase was immobilized on the α-amylase well.

Next, the S1-A well, the S1-B well and the α-amylase well were washed with 200 µL of 1xPBS/T, respectively. Washing was performed 3 times.

Next, bovine serum-derived albumin (produced by Fujifilm Wako Pure Chemical Industries, Ltd., product number 013-15104) was dissolved in 1xPBS/T to prepare a BSA solution. Hereinafter, bovine serum-derived albumin is referred to as BSA. The concentration of BSA in the BSA solution was 3% (w/v).

Next, 200 µL of the BSA solution was added dropwise onto each of the S1-A well, the S1-B well and the α-amylase well, and they were let stand at room temperature for 2 hours. At this time, blocking was performed.

Next, the S1-A well, the S1-B well and the α-amylase well were washed with 200 µL of 1xPBS/T, respectively. Washing was performed 3 times.

Next, 50 µL of the conjugate solution was added dropwise onto each of the S1-A well, the S1-B well and the α-amylase well, and they were let stand at room temperature for 1 hour. The conjugate solution is a solution containing the conjugates synthesized in the above-mentioned "(3-1) Synthesis of conjugate". At this time, in the S1-A well and the S1-B well, some of the conjugates were bound to S1.

Next, the S1-A well, the S1-B well and the α-amylase well were washed with 200 µL of 1xPBS/T, respectively. Washing was performed 3 times. At this time, unbound conjugates that were not bound to the target substances were removed.

Disodium 4-nitrophenyl phosphate hexahydrate (produced by Tokyo Chemical Industry, product number N0241) was prepared. Hereinafter, disodium 4-nitrophenyl phosphate hexahydrate is referred to as pNPP. pNPP is a substrate for alkaline phosphatase-induced metabolism.

Next, pNPP was dissolved in a solution containing a carbonate buffer solution and magnesium sulfate to prepare a pNPP solution. The concentration of pNPP in the pNPP solution was 10 mmol/l. The solution containing the carbonate buffer solution and magnesium sulfate contained 1 mmol/l carbonate buffer solution and 1 mmol/l magnesium sulfate. The pH of the solution containing the carbonate buffer solution and magnesium sulfate was adjusted to 9.6.

The solution dissolving pNPP may be a solution other than the solution containing the carbonate buffer solution and magnesium sulfate. Examples of the solution dissolving pNPP include tris buffer solution, phosphate buffer solution, Good's buffer and the like. The Good's buffer includes MES, Bis-Tris, ADA, PIPES, ACES, MOPSO, BES, MOPS, TES, HEPES, DIPSO, TAPSO, POPSO, HEPPSO, EPPS, Tricine, Bicine, TAPS, CHES, CAPSO, CAPS and the like.

The pH of the solution dissolving pNPP may be a value other than 9.6. The solution dissolving pNPP is preferably an alkalescent solution. The pH of the solution dissolving pNPP preferably falls within a range between 8, inclusive and 11, inclusive. When the solution dissolving pNPP is an alkalescent solution, the change in pH due to a metabolism using pNPP as a substrate becomes large. When the pH of the solution dissolving pNPP falls within a range between 8, inclusive and 11, inclusive, the change in pH due to a metabolism using pNPP as a substrate becomes large.

Next, 100 µL of the pNPP solution was added dropwise onto each of the S1-A well, the S1-B well and the α-amylase well, and they were let stand at 37 °C for 10 minutes. At this time, in the S1-A well and the S1-B well, the enzymatic activity of alkaline phosphatase of the conjugate induces a metabolism using pNPP as a substrate as shown in FIG. 3, and the phosphate group was eliminated from pNPP.

As a result, inorganic phosphoric acid and p-nitrophenol were generated in the S1-A well and the S1-B well. Since the absorption maximum wavelength of p-nitrophenol is 405 nm, yellow color developed in the solution containing p-nitrophenol. In addition, the generated inorganic phosphoric acid was ionized in the solution to release hydrogen ions. Therefore, the pH of the solution decreased.

Next, 5 µL of ethylenediaminetetraacetic acid having a final concentration of 0.5 mol/l was added dropwise onto each of the S1-A well, the S1-B well and the α-amylase well. At this time, the metabolism by the alkaline phosphatase was stopped.

Next, in each of the S1-A well, the S1-B well and the α-amylase well, the absorbance at a wavelength of 405 nm was measured using a microplate reader. Also, the absorbance of the blank was measured in the same manner.

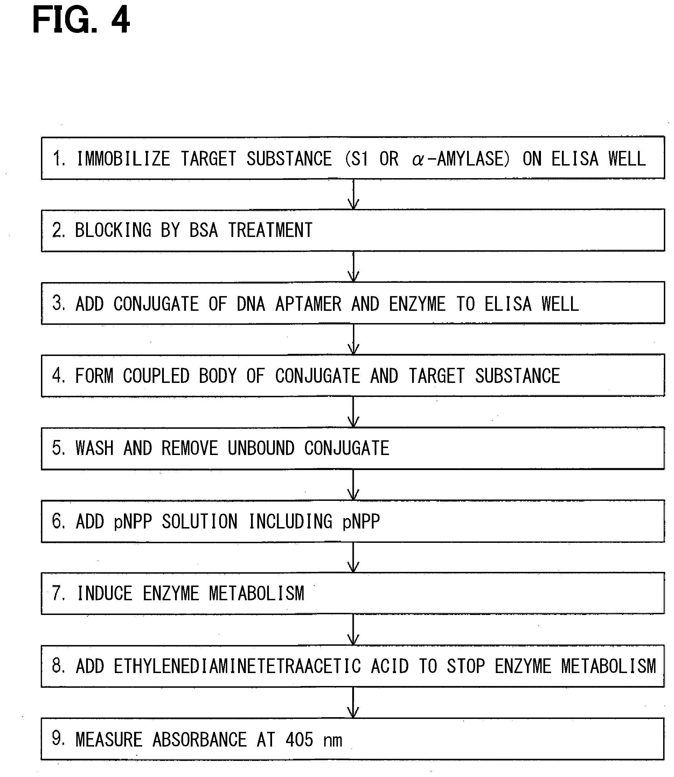

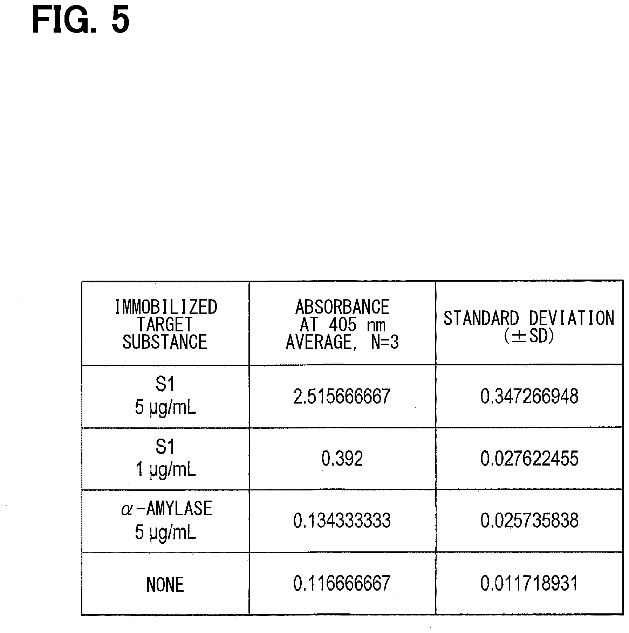

The above process flow is shown in FIG. 4. The measurement results of the absorbance are shown in FIG. 5. "S1 5 µg/mL" in FIG. 5 corresponds to the S1-A well. "S1 1 µg/mL" in FIG. 5 corresponds to the S1-B well. "α-Amylase 5 µg/mL" in FIG. 5 corresponds to the α-amylase well. As shown in FIG. 5, color development was observed only in the S1-A well and the S1-B well.

The measurement results of the absorbance indicate the following. The alkaline phosphatase of the conjugate had an enzymatic activity that induced the metabolism using pNPP as a substrate even in a fused state with the DNA aptamer. In addition, the DNA aptamer of the conjugate had a binding activity to S1 even in the fused state with the label. Moreover, the DNA aptamer of the conjugate did not have a binding activity to α-amylase.

### (3-3) Performing the detection method for the target substance

As a target substance, the novel coronavirus SARS-CoV-2 (hereinafter, referred to as SARS-CoV-2) was prepared. By suspending SARS-CoV-2 in 1xPBS/T, 5 types of suspensions were prepared. The five suspensions differ only in the copy numbers of SARS-CoV-2 contained in the suspensions. The copy numbers of SARS-CoV-2 in the five suspensions are respectively 10¹, 10^{2,} 10³, 10⁴, 10⁵ per 1 µL of the suspension in terms of quantitative PCR. The following steps were performed for each of the five suspensions.

Next, 25 µL of the conjugate solution was added dropwise onto the 25 µL of the suspension, and let them stand at room temperature for 10 minutes. The conjugate solution is a solution containing the conjugates synthesized in the above-mentioned "(3-1) Synthesis of conjugate". The concentration of the conjugate in the conjugate solution was 10 nmol/l. At this time, some of the conjugates are bound to the target substances.

Next, the entire amount of the suspension after being let stand was added dropwise onto the NanoCep centrifugal filtration device with molecular weight cut-off of 300K (manufactured by PALL corporation, product number OD300C34), and high-speed centrifugation was performed. Some of the conjugates that were bound to the target substances were recovered and the remainder of the conjugates that was not bound to the target substances were separated by high-speed centrifugation.

Next, the nanocep centrifugal filtration device with molecular weight cut-off of 300K was washed with 1xPBS/T for 3 times. At this time, the unbound conjugates that were not bound to SARS-CoV-2 were removed.

Next, pNPP was dissolved in a solution containing a carbonate buffer solution and magnesium sulfate to prepare a pNPP solution. The concentration of pNPP in the pNPP solution was 10 mmol/l. The solution containing the carbonate buffer solution and magnesium sulfate contained 1 mmol/l carbonate buffer solution and 1 mmol/l magnesium sulfate. The pH of the solution containing the carbonate buffer solution and magnesium sulfate was adjusted to 9.6.

Next, the pNPP solution was added dropwise onto the NanoCep centrifugal filtration device with molecular weight cut-off of 300K, and it was let stand at 37 °C for 10 minutes. At this time, as shown in FIG. 3, the enzymatic activity of alkaline phosphatase induced the metabolism using pNPP as a substrate, and the phosphate group was eliminated from pNPP. As a result, inorganic phosphoric acid and p-nitrophenol were generated. Since the absorption maximum wavelength of p-nitrophenol is 405 nm, yellow color developed in the solution containing p-nitrophenol. In addition, the generated inorganic phosphoric acid was ionized in the solution to release hydrogen ions. Therefore, the pH of the solution decreased.

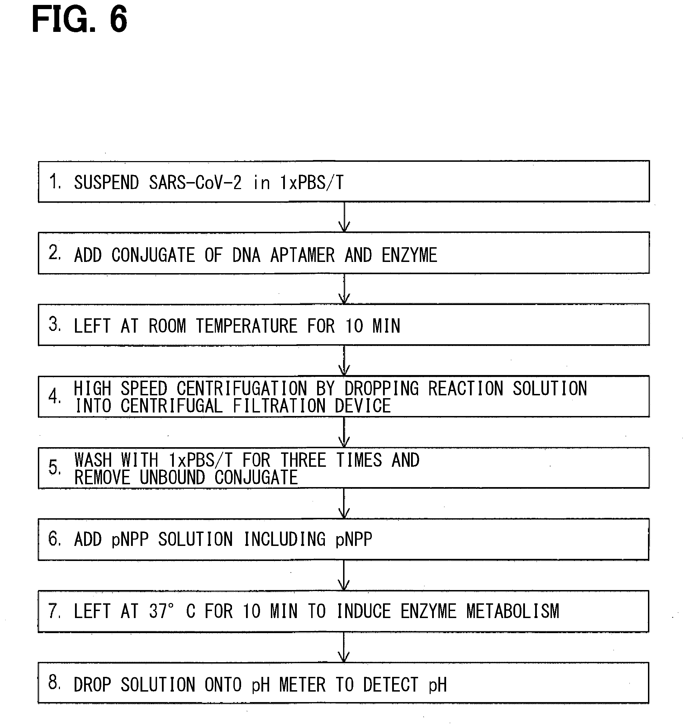

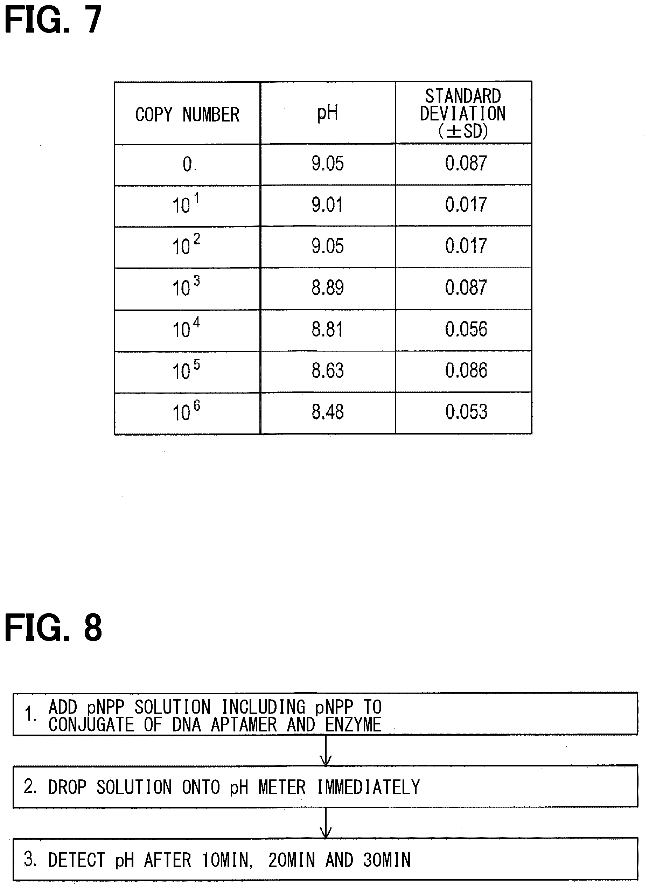

Next, the solution in the nanocep centrifugal filtration device with molecular weight cut-off of 300K was added dropwise onto the pH meter. The pH meter was a compact pH meter LAQUAtwin (produced by HORIBA, Ltd., product number pH-33B). Then, the pH of the solution dropped on the pH meter was recorded. The flow of the above steps is shown in FIG. 6. The pH recording results are shown in FIG. 7. "Copy number" in FIG. 7 means the copy numbers of SARS-CoV-2 per 1µL in the suspension.

As shown in FIG. 7, the greater the copy numbers of SARS-CoV-2 in the suspension, the greater the amount of change in pH. The amount of change in pH means the change amount of pH from the pH of the blank.

The pH recording results indicate the following. The DNA aptamer of the conjugate had a binding activity to SARS-CoV-2 even in the fused state with the label. In addition, the alkaline phosphatase of the conjugate had an enzymatic activity that induces the metabolism using pNPP as a substrate even in the fused state with the DNA aptamer. In addition, inorganic phosphoric acid was generated by the metabolism using pNPP as a substrate and ionized in the solution to release hydrogen ions. As a result, the pH of the solution decreased. Further, when the copy numbers of SARS-CoV-2 in the suspension was 10³ or more, the amount of change in pH was even greater.

In addition, in order to remove the unbound conjugates that were not bound to the target substance, a centrifugal filtration device other than the nanocep centrifugal filtration device with molecular weight cut-off of 300 K may be used. The centrifugal filtration device has, for example, a molecular weight cut-off value equivalent to that of the nanocep centrifugal filtration device with molecular weight cut-off of 300 K.

Further, the method for removing the unbound conjugates that were not bound to the target substance may be a method other than centrifugal filtration. A method for removing the unbound conjugates that were not bound to the target substances may be a sandwich removing method using an ELISA plate or magnetic beads, a removing method by filtration chromatography using a filter paper or a gel, or a removing method by affinity chromatography carrying an antibody at a carrier such as a gel.

Further, the solution dissolving pNPP may be a solution other than the solution containing the carbonate buffer solution and magnesium sulfate. Examples of the solution dissolving pNPP include tris buffer solution, phosphate buffer solution, Good's buffer and the like. The Good's buffer includes MES, Bis-Tris, ADA, PIPES, ACES, MOPSO, BES, MOPS, TES, HEPES, DIPSO, TAPSO, POPSO, HEPPSO, EPPS, Tricine, Bicine, TAPS, CHES, CAPSO, CAPS and the like.

Further, the pH of the solution dissolving pNPP may be a value other than 9.6. The solution dissolving pNPP is preferably an alkalescent solution. The pH of the solution dissolving pNPP preferably falls within a range between 8, inclusive and 11, inclusive. When the solution dissolving pNPP is an alkalescent solution, the change in pH due to the metabolism using pNPP as a substrate becomes large. When the pH of the solution dissolving pNPP falls within a range between 8, inclusive and 11, inclusive, the change in pH due to the metabolism using pNPP as a substrate becomes large.

### (3-4) Test for confirming the expression of the function of the label

By adding the pNPP solution to the conjugate, three kinds of conjugate-pNPP solutions were prepared. The conjugate is synthesized by the above-mentioned "(3-1) Synthesis of conjugate". The pNPP solution was produced in the above-mentioned "(3-2) Using method for detecting the target substance ".

The three conjugate-pNPP solutions differ only in the concentration of the conjugate. The concentrations of the conjugate in the three conjugate-pNPP solutions were 1 pmol/l, 10 pmol/l and 100 pmol/l, respectively.

In each of the three conjugate-pNPP solutions, the alkali phosphatase contained in the conjugate caused the metabolism using pNPP as a substrate. The following steps were performed for each of the three conjugate-pNPP solutions.

Immediately after preparing the conjugate-pNPP solution, the conjugate-pNPP solution was added dropwise onto the pH meter. The pH meter was a compact pH meter LAQUAtwin (produced by HORIBA, Ltd., product number pH-33B).

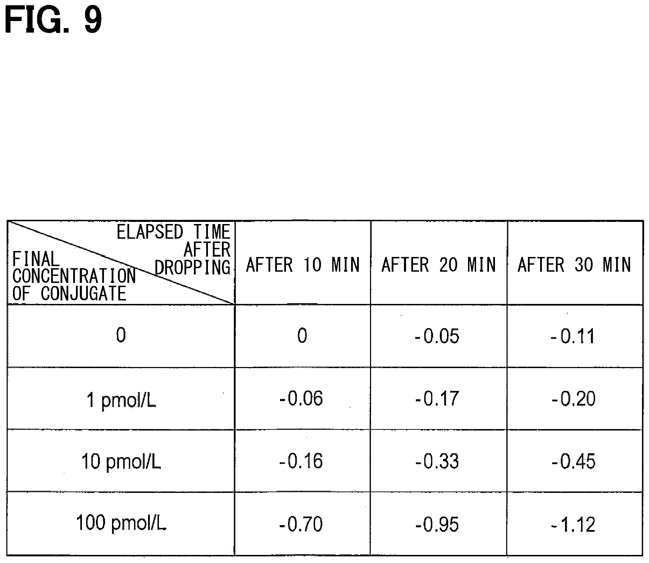

The pH of the conjugate-pNPP solution was recorded 10 minutes after the dropping, 20 minutes after the dropping and 30 minutes after the dropping. The flow of the above steps is shown in FIG. 8. The pH recording results are shown in FIG. 9. The "final concentration of conjugate" in FIG. 9 means the concentration of the conjugate in the conjugate-pNPP solution.

As shown in FIG. 9, the higher the concentration of the conjugate in the conjugate-pNPP solution, the greater the amount of change in pH. The amount of change in pH is the amount of change in pH with respect to pH measured immediately after the dropping.

The pH recording results indicate the following. The alkaline phosphatase contained in the conjugate has an enzymatic activity that induces the metabolism using pNPP as a substrate even in the fused state with the DNA aptamer. In addition, inorganic phosphoric acid generated by the metabolism using pNPP as a substrate is ionized in the solution to release hydrogen ions. As a result, the pH of the solution decreased.

### 4. Other embodiments

Although the embodiments of the present disclosure have been described above, the present disclosure is not limited to the embodiments described above, and various modifications can be made to implement the present disclosure.

(4-1) A plurality of functions of one element in the above embodiment may be implemented by a plurality of elements, or one function of one element may be implemented by a plurality of elements. Further, a plurality of functions of a plurality of elements may be implemented by one element, or one function implemented by a plurality of elements may be implemented by one element. A part of the configuration of the above embodiments may be omitted. Further, at least part of the configuration of the above-described embodiment may be added to or replaced with the configuration of another embodiment described above.

(4-2) In addition to the above-mentioned conjugate, the present disclosure can be realized in various forms such as a member having the conjugate as a component, a method for producing the conjugate, and a method for detecting a target substance and the like.

## Claims

1. A kit of parts for detecting a target substance in a sample comprising a conjugate (7) comprising:
a binding substance (9) having an activity to bind to a target substance (1); and
a label (11) causing a detectable phenomenon, and
a pH meter,
wherein
the target substance is SARS-CoV-2,
the binding substance is a nucleic acid aptamer,
the detectable phenomenon is decrease of pH that can be detected with the pH meter, and
the label is an alkaline phosphatase that metabolises pNPP resulting in the decrease of pH.

## Patentansprüche

1. Kit aus Teilen zum Nachweis einer Zielsubstanz in einer Probe, umfassend ein Konjugat (7), umfassend:
eine Bindungssubstanz (9) mit einer Aktivität, an eine Zielsubstanz (1) zu binden; und
eine Markierung (11), die ein nachweisbares Phänomen verursacht, und ein pH-Meter,
wobei
die Zielsubstanz SARS-CoV-2 ist,
die Bindungssubstanz ein Nukleinsäure-Aptamer ist,
das nachweisbare Phänomen eine pH-Abnahme ist, die mit dem pH-Meter nachgewiesen werden kann, und
die Markierung eine alkalische Phosphatase ist, die pNPP metabolisiert, was zu der pH-Abnahme führt.

## Revendications

1. Trousse de composants pour détecter une substance cible dans un échantillon comprenant un conjugué (7) comprenant :
une substance liante (9) ayant une activité de liaison à une substance cible (1) ; et
un marqueur (11) engendrant un phénomène détectable, et
un pH-mètre,
dans lequel
la substance cible est SARS-CoV-2,
la substance liante est un aptamère d'acide nucléique,
le phénomène détectable est une diminution du pH qui peut être détectée avec le pH-mètre, et
le marqueur est une phosphatase alcaline qui métabolise le pNPP avec pour résultat une diminution du pH.
